# EUROPÄISCHE PATENTSCHRIFT

(11) **EP 2 707 342 B1**
(45) Veröffentlichungstag und Bekanntmachung des Hinweises auf die Patenterteilung: **03.07.2019**
(21) Anmeldenummer: 12720200.0
(22) Anmeldetag: 11.05.2012
(51) Int. Cl.: C04B 41/00, A61K 6/027, A61K 6/02, A61K 6/00, C04B 35/626, C04B 35/628

(54) **VERFAHREN ZUR DOTIERUNG ODER ZUM EINFÄRBEN VON KERAMIK, GLASKERAMIK ODER GLAS**
METHOD FOR DOPING OR COLOURING CERAMIC, GLASS CERAMIC, OR GLASS
PROCÉDÉ DE DOPAGE OU DE COLORATION DE CÉRAMIQUE, DE VITROCÉRAMIQUE OU DE VERRE

(30) Priorität: 13.05.2011 DE 102011101661
(43) Veröffentlichungstag der Anmeldung: 19.03.2014
(73) Patentinhaber: Ivoclar Vivadent AG, 9494 Schaan (LI)
(72) Erfinder: REINSHAGEN, Jörg, 75177 Pforzheim (DE); CRAMER VON CLAUSBRUCH, Sascha, 75417 Lienzingen (DE)
(74) Vertreter: Uexküll & Stolberg
(86) Internationale Anmeldenummer: PCT/EP2012/058818
(87) Internationale Veröffentlichungsnummer: WO 2012/156325

(56) Entgegenhaltungen:
- EP-A2- 1 859 757
- EP-A2- 2 123 185
- DE-A1- 4 207 179
- DE-A1- 19 904 522
- DE-A1-102006 052 030
- DE-A1-102008 026 980

## Beschreibung

Die Erfindung betrifft ein Verfahren zur Dotierung oder zum Einfärben von Keramik, Glaskeramik oder Glas sowie die damit hergestellten Materialien. Weiter betrifft die Erfindung Formkörper, insbesondere dentale Formkörper, die aus den entsprechenden Materialien hergestellt sind. Beschrieben wird Zahnersatz, der wiederum aus solchen Formkörpern hergestellt ist.

Die Einsatzgebiete von Keramiken, insbesondere der sogenannten technischen Keramiken, sind in den letzten Jahren und Jahrzehnten aufgrund ihrer physikalischen und chemischen Eigenschaften immer zahlreicher geworden. Bei den technischen Keramiken handelt es sich dabei um keramische Werkstoffe, die bezüglich ihrer Eigenschaften auf technische Anwendungen hin optimiert wurden. Hier können beispielhaft die Verwendung solcher Keramiken auf dem Dentalgebiet, insbesondere bei der Herstellung von Zahnersatz, oder auch im Schmuckbereich hervorgehoben werden.

Im Dentalbereich werden dabei solche keramischen Materialien nicht nur wie früher für Füllungen und Verblendungen eingesetzt, sondern in letzter Zeit zunehmend auch zur Herstellung vollkeramischer Prothetikteile als Zahnersatz. Dabei wird der Zahnersatz häufig aus einem Formteil, dem sogenannten Rohling oder Blank, der meist aus ungesinterter oder teilgesinterter Keramik besteht, unter Verwendung der CAD/ CAM-Technologie durch mechanische Bearbeitung hergestellt, beispielsweise herausgefräst. Die erhaltenen Zahnprothetikteile werden dann endgesintert und, ggf. nach einer Weiterbearbeitung durch den Zahntechniker, in den Patientenmund eingesetzt.

Nimmt man wiederum das Dentalgebiet als Beispiel für entsprechende Anwendungen von technischen Keramiken, so müssen die verwendeten keramischen Materialien, gerade auch im Hinblick auf die neuen Einsatzmöglichkeiten als Vollkeramik, zum einen im Hinblick auf die geforderten physikalischen oder mechanischen Eigenschaften und zum anderen im Hinblick auf die gewünschte Ästhetik optimiert werden. Verständlicherweise ist es nämlich auch bei vollkeramischen Dentalprothetikteilen, wie beispielsweise bei Dentalgerüsten, erwünscht, dass der Zahnersatz ein möglichst natürliches Aussehen besitzt. In diesem Zusammenhang soll beispielsweise eine gute farbliche Übereinstimmung des keramischen Zahnersatzes mit den verbliebenen natürlichen Zähnen des Patienten erreicht werden. Da die natürliche Zahnfarbe bei verschiedenen Patienten in erheblichem Maße schwankt, ist es dementsprechend erforderlich, eine homogene Einfärbung des Zahnersatzes in verschiedenen Farbtönen bereitzustellen.

Um keramische Formkörper, insbesondere für zahnmedizinische Anwendungen, einzufärben, ist es bereits bekannt, die färbenden Stoffe dem entsprechenden keramischen Werkstoff trocken in Pulverform zuzumischen. Dieses Verfahren beschreibt die WO 2008/023053 A2. Als färbende Stoffe finden Metalloxide, wie Eisenoxid, Verwendung, die dem pulverförmigen keramischen Ausgangsmaterial ebenfalls in Pulverform zugemischt werden.

Aufgrund der Tatsache, dass die färbenden Stoffe als solche in Pulverform eingesetzt werden, bietet sich das Verfahren gemäß der WO 2008/023053 A2 zur Herstellung großer Mengen an eingefärbten Keramikmaterialien an. Die Herstellung kleinerer Mengen an eingefärbter Keramik, insbesondere einer größeren Anzahl an kleineren Chargen mit individuellen Farbtönen, ist in dieser Druckschrift nicht angesprochen.

In der Praxis etabliert sind demgegenüber immer noch die Vorgehensweisen, bei denen ein poröser Formkörper aus Keramik mit Hilfe von Lösungen eingefärbt wird, die Metallionen oder Metallkomplexe enthalten. Bei diesen Formkörpern kann es sich um die genannten Rohlinge oder Blanks, oder insbesondere auch um die aus diesen Rohlingen herausgeformten/herausgefrästen Dentalprothetikteile, handeln.

Dabei wird der poröse Formkörper aus Keramik in der Regel in die Färbelösung eingetaucht, oder die Färbelösung wird mit einem geeigneten Applikator auf die Keramik aufgetragen. Auf diese Weise dringt die Färbelösung teilweise oder vollständig in das poröse Keramikteil ein. Die so behandelte Keramik wird anschließend getrocknet und gesintert, wodurch die endgültige Farbgebung entsteht.

Entsprechende Färbeverfahren sind in der WO 00/46168 A1, der EP 1 486 476 A1 oder auch in der WO 2009/146804 A1 beschrieben.

EP 2 123 185 A2 beschreibt ein Verfahren zur Herstellung von gefärbten Keramikformteilen, bei dem die Oberfläche eines Keramikrohstoffgranulats zumindest teilweise mit einem Farbpigment beschichtet und das beschichtete Keramikrohstoffgranulat anschließend in eine feste Form verpresst wird.

WO 2009/146804 A1 beschreibt ein Verfahren zum Einbringen von Metallionen in poröse Keramiken, bei dem die Keramik mit einer ersten Lösung oder Suspension behandelt wird, die Metallionen und/oder Metallkomplexe enthält, und anschließend mit einer zweiten Lösung oder Suspension behandelt wird, die die in die Keramik eingebrachten Metallionen bzw. Metallkomplexe in der Keramik immobilisiert.

DE 199 04 522 A1 beschreibt ein Verfahren zum Einfärben von Keramiken im porösen oder saugfähigen Zustand unter Verwendung von Metallionen-Lösungen oder Metallkomplex-Lösungen.

DE 10 2006 052 030 A1 beschreibt ein Verfahren zum Herstellen von gefärbten Keramiken, bei dem färbende Metallsalze einem Keramikschlicker zugegeben werden und aus diesem anschließend ein Granulat erzeugt wird.

EP 1 859 757 A2 beschreibt ein Verfahren zur Herstellung von gefärbten Rohlingen und dentalen Formteilen, bei dem ein Oxid-Pulver mit einer farbgebenden Substanz beschichtet wird.

DE 42 07 179 A1 beschreibt ein Verfahren zur Herstellung gesinterter Körper aus Zirkoniumdioxid, bei dem ein durch Copräzipitation und Kalzinierung hergestelltes Pulver, das ZrO₂, ein Stabilisierungsmittel und Verbindungen von Erbium und Praseodym enthält, mit einer Eisenverbindung und einer Zinkverbindung gemischt und die so erhaltene Mischung geformt und gesintert wird.

Die beschriebenen Verfahren, die Färbelösungen verwenden, besitzen jedoch den Nachteil, dass hier jeder einzelne Rohling/Blank oder jedes aus solchen Rohlingen herausgearbeitete Prothetikteil individuell eingefärbt werden muss. Dies ist nicht nur mit einem erheblichen Aufwand für den Anwender, in der Regel den Zahntechniker, verbunden. Darüber hinaus lassen sich dabei Abweichungen in der endgültigen Farbe verschiedener Teile, trotz sorgfältiger Einhaltung aller Verfahrensparameter, nicht immer vermeiden.

Die Erfindung stellt sich dementsprechend die Aufgabe, die geschilderten und weitere Nachteile des beschriebenen Standes der Technik zu vermeiden. Insbesondere soll ein Verfahren zur Einfärbung von Keramik, Glaskeramik oder Glas bereitgestellt werden, das nicht nur für vergleichsweise große, sondern auch für kleinere Materialmengen eine vergleichsweise einfache Einfärbung mit reproduzierbaren Ergebnissen möglich macht. Auf diese Weise soll erreicht werden, dass dem Anwender, beispielsweise dem Zahntechniker, problemlos Formkörper, wie beispielsweise Rohlinge, mit verschiedenen Farbtönen zur Verfügung gestellt werden können.
Dabei soll das Verfahren so ausgestaltet sein, dass es grundsätzlich nicht auf die Einfärbung der genannten Materialien beschränkt ist, sondern dass diese Materialien auch gezielt mit zusätzlichen Elementen, die also nicht zwangsläufig eine färbende Wirkung besitzen, jedoch eine Änderung der Materialeigenschaften für den gewünschten Anwendungszweck bewirken, dotiert werden können.

Diese Aufgabe wird gelöst durch das Verfahren mit den Merkmalen des Anspruchs 1. Bevorzugte Ausführungen dieses Verfahren sind in den abhängigen Ansprüchen 2 bis 10 beschrieben. Anspruch 11 definiert das Granulat, das mit dem beanspruchten Verfahren erhältlich ist. Die Ansprüche 12 und 13 definieren die Formkörper, die aus den erfindungsgemäßen Materialien hergestellt sind.
Der Wortlaut sämtlicher Ansprüche wird hiermit durch Bezugnahme zum Inhalt dieser Beschreibung gemacht.

Das erfindungsgemäße Verfahren der eingangs genannten Art ist insbesondere bei der Herstellung von Formkörpern, die mindestens teilweise aus Keramik, Glaskeramik oder Glas bestehen, vorgesehen. Bei diesem Verfahren wird die Keramik, die Glaskeramik oder das Glas als Granulat bereitgestellt und dieses Granulat wird dann mit einer Lösung, die Metallionen und/oder Metallkomplexe enthält, besprüht.

In diesem Zusammenhang sollen die im Zusammenhang mit Anspruch 1 verwendeten Begriffe wie folgt erläutert werden:
Unter "Dotierung" soll das vorzugsweise gezielte Einbringen von Metallen bzw. Metallionen in die erfindungsgemäß verwendeten Materialien (Keramik, Glaskeramik, Glas) verstanden werden. Dieses Einbringen wird dabei in der Regel so erfolgen, dass die Metallatome oder Metallionen Plätze einnehmen, die zuvor von anderen Atomen besetzt waren, oder dass sie sich zwischen solchen Plätzen anordnen. Bei kristallinen oder teilkristallinen Materialien können diese Plätze dementsprechend Gitterplätze oder auch Zwischengitterplätze sein. Alternativ können sich die Metallatome/Metallionen auch an Korngrenzen anlagern oder innerhalb des Materials, beispielsweise bei Glas, Ausscheidungen, insbesondere nanopartikuläre bzw. kolloidale Ausscheidungen bilden.

Besitzt das eingebrachte Metallatom oder Metallion eine färbende Wirkung oder entfaltet es eine solche färbende Wirkung im Material, so soll erfindungsgemäß von einem "Einfärben" die Rede sein.

Unter "Keramik" werden erfindungsgemäß anorganische, nicht metallische Werkstoffe verstanden, die weitgehend kristallin, d.h. aus Kristalliten/Körnern aufgebaut sind. Eine "Glaskeramik" weist in der Regel in einer amorphen (Glas-)Matrix kristalline (keramische) Bereiche auf. "Glas" ist ein amorpher, nicht kristalliner Feststoff. Es handelt sich dabei um eine amorphe Substanz, die thermodynamisch als gefrorene, unterkühlte Flüssigkeit bezeichnet werden kann.

Bei "Granulat" soll es sich um ein Material handeln, bei dem mehrere oder viele Körner/Kristallite zu größeren Partikeln zusammengelagert sind, in der Regel mit Hilfe von Bindemitteln, insbesondere organischen Bindemitteln. Diese (größeren) Partikel sind vorzugsweise gleichförmig, z.B. ellipsoid oder insbesondere kugelförmig aufgebaut. Zwischen diesen Partikeln befinden sich Poren, die auch die Form engerer, langgestreckter Kapillaren aufweisen können. Auch innerhalb der Partikel selbst können sich solche Poren befinden.

Granulate sind, insbesondere im trockenen Zustand, in der Regel rieselfähig und können durch verschiedene Verfahren, die dem Fachmann bekannt sind, hergestellt werden, beispielsweise durch Sprühgranulation.

Der verwendete Ausdruck "Lösung" ist dem Fachmann ohne weiteres bekannt und soll hier möglichst umfassend verstanden werden. Eine (flüssige) Lösung ist das homogene Gemisch eines entsprechenden Feststoffs in mindestens einem Lösungsmittel.

Wie oben ausgeführt ist ein entscheidendes Merkmal des erfindungsgemäßen Verfahrens, dass die Keramik, die Glaskeramik oder das Glas als Granulat (!), d.h. als ein bereits zu größeren Partikeln zusammengelagertes Material mit einer Lösung, die Metall-ionen oder Metallkomplexe enthält, besprüht wird.

Von Bedeutung ist, dass die Metallionen oder Metallkomplexe, die in der Lösung enthalten sind, möglichst gleichmäßig auf dem Granulat bzw. im Granulat verteilt werden. Auf diese Weise ist dann gewährleistet, dass auch im resultierenden Formkörper die dotierenden und/oder farbgebenden Metallionen möglichst gleichmäßig verteilt sind.

Erfindungsgemäß wird das Granulat mit der Lösung besprüht. Damit wird zum einen eine möglichst gleichmäßige Verteilung der Metallionen auf/im Granulat erreicht. Zum anderen wird bei einem solchen Sprühvorgang die Lösung nur in der Menge aufgebracht/eingebracht, die für die Dotierung/Einfärbung tatsächlich notwendig ist. Die Lösung wird also sparsam verwendet.

Häufig ist das erhaltene behandelte Granulat direkt weiterverarbeitbar, insbesondere als rieselfähiges oder fließfähiges Produkt, ggf. mit einer (Rest-)Feuchte. Diese Restfeuchte beträgt in der Regel weniger als 20 %, insbesondere zwischen 5 und 15 %.

In Weiterbildung kann es auch von Vorteil sein, wenn das mit der Lösung behandelte Granulat (nach dem Besprühen) getrocknet wird. Auf diese Weise wird das für die Herstellung der Lösung verwendete Lösungsmittel oder Lösungsmittelgemisch vor der Weiterverarbeitung des behandelten Granulats im größeren Maße entfernt. Dies kann insbesondere wichtig sein, wenn eine solche Weiterverarbeitung trocken, beispielsweise durch Trockenpressen, erfolgen soll.

Im übrigen ist es auch möglich, das Granulat vor der Behandlung mit der Lösung in einem separaten Schritt zu trocknen.

Für die beschriebene Trocknung werden in der Regel Temperaturen unter 150 °C, insbesondere unter 100 °C, ausreichen. Solche Temperaturen sind geeignet, übliche Lösungsmittel, beispielsweise die im folgenden noch beschriebenen Alkohole, von dem Granulat zu entfernen.

Bei weiteren bevorzugten Ausführungsformen des erfindungsgemäßen Verfahrens wird das Granulat beim Besprühen mit der Lösung, bewegt. Bei diesem Bewegen kann es sich beispielsweise um ein Umwälzen oder Umrühren des Granulats handeln. Dies lässt sich beispielsweise mit Hilfe von Luft oder eines Gases, wie Stickstoff oder Argon, oder mit Hilfe einer geeigneten Rühreinrichtung erreichen.

Alternativ kann das Bewegen, insbesondere Umwälzen oder Umrühren, auch erst bei dem zusätzlichen Trocknungsschritt erfolgen. Es ist auch möglich und vorteilhaft, dieses Bewegen sowohl beim Besprühen als auch beim Trocknen anzuwenden.

Die Bewegung beim Besprühen verbessert die Verteilung der Metallionen aus der Lösung auf oder im Granulat. Die Bewegung beim Trocknen unterstützt das Entfernen des Lösungsmittels beim Trocknungsvorgang.

Weiter ist es bei dem erfindungsgemäßen Verfahren bevorzugt, wenn die Lösung Metallionen oder Metallkomplexe der Seltenerdenelemente oder der Nebengruppenelemente des Periodensystems der Elemente enthält. Bekanntlich sind von den Seltenerdenelementen insbesondere auch die Gruppe der Lanthanoide umfasst.

Bei den Nebengruppenelementen sind insbesondere die Übergangsmetalle aus den Nebengruppen III B, IV B, V B, VI B, VII B, VIII B, I B, II B hervorzuheben. Unter Zugrundelegung der neueren Nomenklatur des Periodensystems der Elemente handelt es sich hier also um die Gruppen 3, 4, 5, 6, 7, 8, 11 und 12.

Besonders bevorzugt einsetzbar sind bei dem erfindungsgemäßen Verfahren Lösungen, die Metallionen oder Metallkomplexe mit mindestens einem der Elemente Eisen (Fe), Chrom (Cr), Kupfer (Cu), Yttrium (Y), Praseodym (Pr), Kobalt (Co), Nickel (Ni), Mangan (Mn), Erbium (Er) oder Cer (Ce) enthalten.

Mit Vorteil sind bei dem erfindungsgemäßen Verfahren in der Lösung, die zur Dotierung oder Einfärbung vorgesehen ist, Metallionen oder Metallkomplexe der Erdalkalimetalle, insbesondere von Calcium und/oder Magnesium, enthalten. Alternativ oder zusätzlich können auch Aluminiumionen oder Aluminiumkomplexe enthalten sein. Auf diese Weise können die Materialien (Keramik, Glaskeramik, Glas) auch mit Erdalkalimetallen bzw. mit Aluminium dotiert werden.

Die genannten Lösungen lassen sich erfindungsgemäß in der Regel dadurch herstellen, dass ein entsprechendes Metallsalz in dem entsprechenden Lösungsmittel oder Lösungsmittelgemisch aufgenommen, insbesondere aufgelöst, wird. Vorzugsweise wird bei der Erfindung dabei von den entsprechenden Salzen, wie Halogeniden, insbesondere Chloriden, Sulfaten, Carbonaten oder insbesondere Nitraten des jeweiligen Metalls ausgegangen.

Ggf. können in der Lösung (zusätzliche) Komplexbildner enthalten sein, die dann die entsprechenden Komplexverbindungen mit den Metallen oder Metallionen ausbilden. Dabei kann es sich um die dem Fachmann bekannten anorganischen Komplexbildner oder die bekannten organischen Komplexbildner, wie beispielsweise Acetylaceton, EDTA (Ethylendiamintetraacetat) oder NTA (Nitrilotriessigsäure) handeln.

Als Lösungsmittel zur Herstellung der verwendeten Lösungen können grundsätzlich die verschiedensten Lösungsmittel, insbesondere polaren Lösungsmittel, eingesetzt werden. Hierbei kann es sich u.a. um polare organische Lösungsmittel, wie aliphatische Alkohole, insbesondere Methanol, Ethanol u.a., handeln. Insbesondere kann aber auch als Lösungsmittel Wasser allein oder Wasser in Mischung mit organischen polaren Lösungsmitteln, vorzugsweise Alkoholen, eingesetzt werden. Ggf. können in der Lösung neben den genannten Komplexbildnern auch noch sogenannte Stabilisatoren, Dispergiermittel u.dgl. enthalten sein.

Bei der Erfindung ist die Konzentration der Metallionen bzw. Metallkomplexe in der Lösung innerhalb weiter Grenzen variierbar. Entscheidend ist, dass das Inkontaktbringen des Metall-ions/Metallkomplexes mit dem Granulat in Lösung, d.h. zusammen mit dem entsprechenden Lösungsmittel, erfolgt. Auf diese Weise wird, wie erläutert, eine gleichmäßige Verteilung der zur Dotierung oder Einfärbung vorgesehenen Metallionen auf oder im Granulat gewährleistet.

Vorzugsweise sind die Metallionen oder Metallkomplexe in der Lösung in einer Konzentration zwischen 0,01 Gew.-% und 70 Gew.-%, vorzugweise zwischen 0,1 Gew.-% und 50 Gew.-%, enthalten. Innerhalb des zuletzt genannten Bereichs sind insbesondere Konzentrationen zwischen 2 Gew.-% und 25 Gew.-% hervorzuheben.
Je nach färbender Wirkung der einzelnen Metallionen sind entweder geringere Mengen an Metallionen ausreichend oder größere Mengen an Metallionen erforderlich. Dabei versteht es sich, dass je nach zu erzielendem Farbton auch mindestens zwei verschiedene Metallionen in der Lösung enthalten sein können. Dies wird dadurch erreicht, dass mindestens zwei Metallsalze, ggf. unter Zugabe von Komplexbildnern, in dem entsprechenden Lösungsmittel oder Lösungsmittelgemisch gelöst werden.

Erfindungsgemäß ist es auch möglich, die insgesamt zur Dotierung oder Einfärbung verwendete Menge an Lösung auf die Menge an Pulver zu beziehen, die mit der Lösung behandelt wird. Auch hier ist eine Variation der Mengenverhältnisse innerhalb breiter Grenzen möglich. Hervorzuheben sind dabei Mengen zwischen 0,0001 -1 g Lösung pro g Granulatmenge, insbesondere 0,001 - 0,5 g Lösung pro g Granulatmenge.

Vorzugsweise beträgt der Anteil der Lösung im behandelten Material ≤ 20 Gew.%, insbesondere ≤ 15 Gew.%. Unterhalb der genannten Obergrenzen, insbesondere bei Anteilen zwischen 5 und 15 Gew.%, werden nicht zu feuchte behandelte Granulate erhalten, die als rieselfähige Produkte direkt durch Trockenpressen zu Rohlingen weiterverarbeitet werden können.

Wie oben erläutert, wird das zu dotierende Material bzw. das einzufärbende Material als Granulat eingesetzt. Die mittlere Partikelgröße dieses Granulats (D50) liegt vorzugsweise zwischen 1 µm und 200 µm, insbesondere zwischen 5 µm und 100 µm. Das Granulat wiederum kann aus Pulvern mit einer mittleren Partikelgröße (D50) zwischen 1 nm und 50 µm, vorzugsweise zwischen 10 nm und 10 µm aufgebaut sein. Innerhalb des letztgenannten Bereichs sind mittlere Partikelgrößen zwischen 100 nm (0,1 µm) und 1 µm hervorzuheben.

Erfindungsgemäß wird das Granulat nach dem Besprühen mit der Lösung und ggf. nach dem sich daran anschließenden Trocknen vorzugsweise direkt zu einem Formkörper weiterverarbeitet. Die Bereitstellung des Formkörpers kann dabei auf die unterschiedlichste Weise erfolgen. Insbesondere wird es sich hierbei um einen Pressvorgang, insbesondere um einen Trockenpressvorgang, handeln.

Bei solchen Pressvorgängen bzw. Pressschritten wird das keramische, glaskeramische oder Glas-Material, d.h. das erfindungsgemäß behandelte Material, unter Anwendung eines hohen Drucks in eine Pressform hineingepresst. Dieses Pressen kann entweder nur aus einer Richtung axial (uniaxial) oder aus beiden Richtungen axial (biaxial) erfolgen. Auch eine isostatische Pressung ist möglich. Hier unterscheidet man zwischen einer vollisostatischen Pressung, bei der eine Druckbeaufschlagung gleichzeitig aus allen Richtungen mit gleichem Pressdruck erfolgt, und einer quasi-isostatischen Pressung, bei der bei einer zylindrischen Pressform gleichzeitig biaxial und radial gepresst wird. Je nach Material und Pressdrucken können die gewünschten Gefügestrukturen und Festigkeiten mit allen Pressarten erreicht werden.

Zur endgültigen Fertigstellung des Formkörpers kann nach dem Pressvorgang in der Regel ein Entfernen (Entbindern) des vorhandenen organischen Bindemittels oder Presshilfsmittels erfolgen. Dann erfolgt, insbesondere bei keramischen Materialien, ein Ansintern bei höherer Temperatur, um die Porosität des Materials weiter zu verringern. In der Regel wird jedoch hier nicht endgesintert, um nachfolgend eine leichtere mechanische Bearbeitbarkeit des Materials zu gewährleisten.

Wird im vorliegenden Fall eine Keramik dotiert bzw. eingefärbt, handelt es sich vorzugsweise um eine Keramik auf Basis von Zirkonoxid oder Aluminiumoxid. Besonders hervorzuheben sind hier die eingangs erwähnten sogenannten technischen Keramiken, die dem Fachmann ohne weiteres bekannt sind.

Werden Dentalkeramiken behandelt, so handelt es sich hier vorzugsweise um Keramiken, die Bestandteile wie Zirkonoxid, Yttriumoxid, Hafniumoxid, Aluminiumoxid und ggf. weitere Oxide umfassen.

Werden erfindungsgemäß Gläser behandelt, so handelt es sich hier vorzugsweise um Silikatgläser oder um oxidische Gläser und dabei insbesondere um Borosilikatgläser oder Alumosilikatgläser. Besonders hervorzuheben sind Alkaliborosilikatgläser, da sich diese besonders gut als amorphe, poröse Festkörper darstellen lassen. Definitionsgemäß besitzen solche Alkaliborosilikatgläser als Bestandteile Alkalimetalloxide, in der Regel Natriumoxid (Na₂O) und/oder Kaliumoxid (K₂O) und Bortrioxid (B₂O₃). Weitere Bestandteile, in der Regel Hauptbestandteile, sind Siliziumdioxid (SiO₂) und Aluminiumoxid (Al₂O₃).

Zusammenfassend liegt der Kern der vorliegenen Erfindung darin, dass eine Dotierung, insbesondere Einfärbung des entsprechenden Materials, insbesondere einer Dentalkeramik, an einem granulierten Material mit Hilfe einer Lösung, die Metallionen und/oder Metallkomplexe enthält, erfolgt. Dementsprechend werden keine pulverförmigen Materialien miteinander vermischt und auch kein pulverförmiges Material mit einer Lösung, sondern ein granuliertes Ausgangsmaterial wird mit der (Färbe-)Lösung erfindungsgemäß in Kontakt gebracht. Dieses Ausgangsmaterial ist aufgrund der in ihm vorhandenen Poren und Kapillaren saugfähig und dementsprechend von der Lösung gut benetzbar, so dass die Lösung gut in die vorhandenen Poren/Kapillaren eindringen und diese füllen, insbesondere im wesentlichen vollständig füllen kann.

Die Erfindung ordnet sich dementsprechend beispielsweise in die Prozesskette für die Herstellung eines dentalen Formteils wie folgt ein.

Es wird zunächst ein Granulat aus dem entsprechenden Material, beispielsweise der Dentalkeramik hergestellt oder bereitgestellt. Dieses Granulat wird mit der (Färbe-)Lösung besprüht (und gemischt). Auf diese Weise wird ein (eingefärbtes) Granulat erhalten, das in der Regel noch feucht ist und direkt als fließfähiges Material zu einem entsprechenden Rohling verpresst werden kann.

Dabei ist hervorzuheben, dass erfindungsgemäß ein mit der Lösung behandeltes Granulat bereitgestellt wird, bei dem die (Färbe-)Lösung im wesentlichen in den Poren bzw. in den Kapillaren, d.h. im Inneren der Granulatteilchen gespeichert ist. Dementsprechend ist das behandelte Granulat, makroskopisch betrachtet, von außen her im wesentlichen trocken und behält auf diese Weise die wesentlichen Eigenschaften eines (trockenen) Granulats, d.h. seine Fließfähigkeit und seine Fähigkeit, trocken zu einem Formkörper verpresst zu werden.

Ein Trocknen des behandelten Granulats vor dem Verpressen ist lediglich optional. Optional kann das behandelte (eingefärbte) Granulat vor dem Verpressen entweder mit ungefärbtem Granulat oder mit anders eingefärbten Granulaten vermischt werden, um entweder hellere Farbtöne oder Misch-Farbtöne zu erhalten. Für den Pressvorgang enthält das behandelte Granulat in der Regel zusätzlich einen üblicherweise eingesetzten (bekannten), beispielsweise organischen Binder. Dieser Binder wird nach dem Pressen aus dem erhaltenen Rohling durch eine Wärmebehandlung (Entbindern) entfernt, und der Rohling in der Regel angesintert, jedoch nicht endgesintert. Auf diese Weise ist der Rohling in einfacher Weise, beispielsweise durch Fräsen mechanisch bearbeitbar, um daraus Zahnersatzteile, wie Gerüste für Kronen und Brücken, Verblendungen oder auch fertige Keramikteile wie Kronen und Brücken zu erhalten. Der so erhaltene Zahnersatz wird in einem Ofen dicht- bzw. endgesintert, so dass auf diese Weise ein eingefärbter Zahnersatz erhalten werden kann, der vom Zahntechniker und vom Zahnarzt in üblicher Weise endbearbeitet und in den Mund des Patienten eingesetzt wird.

Die Erfindung umfasst darüber hinaus die Keramik, die Glaskeramik oder das Glas, welche nach dem beschriebenen erfindungsgemäßen Verfahren herstellbar oder hergestellt sind. Insbesondere handelt es sich bei dem genannten Material um eine Keramik, vorzugsweise für dentale Anwendungen um eine Feldspatkeramik oder Lithiumdisilikatkeramik.

Von der Erfindung auch umfasst ist ein Formkörper, insbesondere ein dentaler Formkörper, der aus dem erfindungsgemäßen Material (Keramik, Glaskeramik, Glas) hergestellt ist. Bei diesem Formkörper handelt es sich insbesondere um einen ungesinterten oder nicht endgesinterten Rohling für die Herstellung von Zahnersatz, vorzugsweise von vollkeramischem Zahnersatz. Dieser Formkörper ist vorzugsweise, beispielsweise aufgrund seiner Porosität, einfach mechanisch bearbeitbar, insbesondere durch Fräsen, so dass auf diese Weise der Zahnersatz, beispielsweise in Form von Gerüsten, Kronen, Brücken u.dgl., gefertigt werden kann.

Bei dem genannten Formkörper handelt es sich vorzugsweise um einen sogenannten Rohling oder Blank mit grundsätzlich beliebiger Geometrie. Solche Rohlinge können quaderförmig, würfelförmig oder zylinderförmig sein. Auch beliebige andere Geometrien, wie beispielsweise kegelförmige oder kugelförmige Rohlinge sind möglich. Insbesondere bei zylinderförmigen Rohlingen kann es sich vorzugsweise um scheibenförmige Rohlinge handeln. Dabei ist eine Scheibe definitionsgemäß ein Körper, insbesondere Zylinder, dessen Dicke wesentlich geringer ist als sein Radius. Die Scheibe ist vorzugsweise rund, kann jedoch auch andere Geometrien aufweisen, beispielsweise eine Form, die der Silhouette eines Hufeisens im wesentlichen entspricht.

Schließlich umfasst die vorliegende Beschreibung auch den Zahnersatz, beispielsweise das Gerüst, die Krone oder die Brücke oder auch die Verblendung, die aus dem beanspruchten erfindungsgemäßen Formkörper hergestellt ist.

Die Erfindung ist mit einer ganzen Reihe von Vorteilen verbunden.

Aufgrund der Ausgestaltung des erfindungsgemäßen Verfahrens können Nachteile der eingangs beschriebenen bekannten Verfahren vermieden werden. Aufgrund der Tatsache, dass nicht jeder einzelne Rohling oder jedes einzelne gefertigte Zahnersatzteil eingefärbt werden muss, lassen sich mögliche Verfahrensfehler vermeiden. Dem Zahntechniker wird ein reproduzierbar eingefärbter Rohling an die Hand gegeben, aus dem er mehrere Zahnersatzteile mit konstanter Farbgebung fertigen kann.

Andererseits ist es nicht notwendig, die färbenden Metallionen als Pulver, beispielsweise als Metalloxide, einem Keramikpulver zuzugeben.

Aufgrund der Tatsache, dass die Metallionen über eine Lösung mit dem Ausgangsmaterial, das als Granulat vorliegt, in Kontakt gebracht werden, lassen sich auch kleinere Mengen an Ausgangsmaterial zuverlässig und reproduzierbar mit den Metallionen dotieren oder einfärben. Es können also nach Bedarf auch kleine Serien an Rohlingen mit einer bestimmten Farbgebung produziert werden. Dies erlaubt die Dotierung bzw. Einfärbung einerseits nah am Ausgangsmaterial, wobei andererseits die Nachteile des Einfärbens am fertigen Produkt, wie es derzeit überwiegend gehandhabt wird, vermieden werden können.

Ein weiterer Vorteil ist, dass sich eine Formgebung des erfindungsgemäß behandelten Materials, beispielsweise zu den genannten Rohlingen, unmittelbar an die Dotierung/Einfärbung anschließen kann. Weitere Zwischenschritte wie ein Calcinieren (Erhitzen), Granulieren oder eine sonstige Aufbereitung sind nicht nötig.

Die geschilderten und weitere Merkmale der Erfindung ergeben sich aus dem nachfolgenden Beispiel in Verbindung mit den Unteransprüchen. Dabei können die einzelnen Merkmale für sich allein oder in Kombination miteinander verwirklicht sein.

### Beispiel

Es wird von einem kommerziell erhältlichen Granulat ausgegangen, das wie folgt herstellt werden kann.

Zunächst wird ein Pulver zur Herstellung einer Dentalkeramik mit der folgenden Zusammensetzung bereitgestellt:

| **Bestandteil** | **Formel** | **Gew.-%** |
|---|---|---|
| Yttriumoxid | Y₂O₃ | 5,36 |
| Aluminiumoxid | Al₂O₃ | 0,05 |
| Zirkonoxid + | ZrO₂ + | > 99,0 |
| Yttriumoxid + | Y₂O₃ + | |
| Hafniumoxid | HfO₂ | |
| Andere Oxide | | Rest |

Eine solche Zusammensetzung ist auch unter der Bezeichnung 3Y-TZP bekannt (mit 3 Mol.% Yttriumoxid teilstabilisiertes tetragonales Zirkoniumoxid).

1 kg der oben genannten Pulvermischung wird zusammen mit einem Anteil von 3,8 Gew.-% eines organischen Bindemittels, das später auch als Presshilfsmittel dient, sprühgranuliert. Vor der Sprühgranulation beträgt die mittlere Partikelgröße (D50) des Pulvers 0,43 µm. Nach dem Granulieren beträgt die mittlere Partikelgröße des Granulats 52 µm (D10 = 35 µm, D50 = 52 µm, D90 = 90 µm).

Parallel wird eine Färbelösung aus 30 g Eisen(III)nitrat-Nonahydrat in 80 g demineralisiertem Wasser hergestellt. Das Eisensalz löst sich im Wasser vollständig auf.

Das oben genannte Granulat (1 kg) wird in eine Apparatur im Labormaßstab eingebracht, die neben einem Behälter mit Rühreinrichtung eine Sprühdüse zum Aufbringen der Lösung des Metallsalzes auf das Granulat aufweist. Das im Behälter vorgelegte Granulat wird mit Hilfe der Rühreinrichtung in eine gleichmäßige Bewegung versetzt. Bekanntlich ist das Granulat als Schüttgut fließfähig. Dann wird die Lösung des Metallsalzes mit Hilfe der Sprühdüse als Sprühnebel über dem Keramikgranulat ausgetragen und auf diese Weise mit dem Granulat in Kontakt gebracht. Es ist auch möglich, die Lösung direkt in das bewegte und/oder verwirbelte Granulat, in der Regel unter Druck, fein verteilt, d.h. vernebelt, einzubringen, insbesondere einzusprühen.

Aufgrund der Tatsache, dass das Granulat mit Hilfe der Rühreinrichtung bewegt, d.h. umgewälzt, wird, kommt im Laufe der Zeit die gesamte Oberfläche der Granulatpartikel mit der Lösung in Berührung. Man kann auch davon sprechen, dass die Oberfläche der Granulatpartikel im wesentlichen gleichmäßig mit der Lösung benetzt wird. Die Lösung kann auf diese Weise insbesondere auch in vorhandene Poren in den Granulatpartikeln und zwischen den Granulatpartikeln eindringen.

Im Beispiel wird die gesamte oben angegebene Lösung, die aus 80 g demineralisertem Wasser und 30 g Eisen(III)nitrat-Nonahydrat besteht auf 1 kg Granulat aufgebracht. Die aufgebrachte Menge an Lösung beträgt dabei also ca. 10 Gew.%, bezogen auf die Gesamtmenge an vorgelegtem Granulat.

Das eingefärbte feuchte Granulat wurde dann ohne vorherige Trocknung in einem uniaxialen (Trocken-) Pressvorgang mit einer Presskraft von 1200 kN zu einem scheibenförmigen Grünkörper (Rohling) mit 100 mm Durchmesser und 16 mm Dicke verpresst. Die so erhaltenen Rohlinge wurden bei 1000 °C angesintert und dienten dann zur Herstellung von Zahnersatz. So wurden beispielsweise aus angesinterten Rohlingen Kronen gefräst, die eine gute Festigkeit und eine homogene Farbverteilung aufwiesen.

Die Vorgehensweise gemäß Beispiel wurde auf verschiedene Weise abgewandelt. So wurden andere Metallsalze zur Herstellung von Färbelösungen verwendet, und zwar in unterschiedlichen Konzentrationen. Außerdem wurden die gemäß Beispiel erhaltenen eingefärbten Granulate mit nicht eingefärbtem Granulat vermischt, um hellere Farbtöne zu erhalten. Dieser Mischvorgang wurde beispielsweise mit Hilfe eines Rhönradmischers vorgenommen, wie er üblicherweise zum Mischen granulatförmiger Produkte verwendet wird.

## Patentansprüche

1. Verfahren zur Dotierung oder zum Einfärben von Keramik, Glaskeramik oder Glas, insbesondere bei der Herstellung von Formkörpern, die mindestens teilweise aus diesen Materialien bestehen, wobei die Keramik, die Glaskeramik oder das Glas als Granulat bereitgestellt wird und dieses Granulat mit einer Lösung, die Metallionen und/oder Metallkomplexe enthält, besprüht wird, wobei in dem Granulat Poren und/oder Kapillaren vorhanden sind und die Lösung in diese Poren und/oder Kapillaren eindringt.

2. Verfahren nach Anspruch 1, **dadurch gekennzeichnet, dass** das mit der Lösung behandelte Granulat getrocknet wird, vorzugsweise bei einer Temperatur < 100 °C.

3. Verfahren nach einem der vorhergehenden Ansprüche, **dadurch gekennzeichnet, dass** das Granulat beim Besprühen und/oder beim Trocknen bewegt, insbesondere umgewälzt oder umgerührt, wird.

4. Verfahren nach einem der vorhergehenden Ansprüche, **dadurch gekennzeichnet, dass** in der Lösung Metallionen oder Metallkomplexe der Seltenerdenelemente, insbesondere der Lanthanoide, oder der Nebengruppenelemente des Periodensystems der Elemente enthalten sind.

5. Verfahren nach Anspruch 4, **dadurch gekennzeichnet, dass** in der Lösung Metallionen oder Metallkomplexe mindestens eines der Elemente Eisen, Chrom, Kupfer, Yttrium, Praseodym, Kobalt, Nickel, Mangan, Erbium oder Cer enthalten sind.

6. Verfahren nach einem der vorhergehenden Ansprüche, **dadurch gekennzeichnet, dass** die Lösung mindestens ein polares Lösungsmittel aufweist, wobei es sich bei dem polaren Lösungsmittel vorzugsweise um Wasser oder mindestens einen aliphatischen Alkohol handelt.

7. Verfahren nach einem der vorhergehenden Ansprüche, **dadurch gekennzeichnet, dass** die Metallionen oder Metallkomplexe in der Lösung in einer Konzentration zwischen 0,01 Gew.-% und 70 Gew.-%, insbesondere zwischen 0,1 Gew.-% und 50 Gew.-%, enthalten sind.

8. Verfahren nach einem der vorhergehenden Ansprüche, **dadurch gekennzeichnet, dass** das Granulat nach dem Besprühen und ggf. nach dem Trocknen zu einem Formkörper weiterverarbeitet wird, vorzugsweise mit Hilfe mindestens eines Pressschritts, insbesondere Trockenpressschritts.

9. Verfahren nach einem der vorhergehenden Ansprüche, **dadurch gekennzeichnet, dass** es sich bei dem Material um eine Keramik, vorzugsweise um eine Keramik auf Basis von Zirkonoxid (ZrO₂) oder Aluminiumoxid (Al₂O₃), handelt.

10. Verfahren nach Anspruch 9, **dadurch gekennzeichnet, dass** es sich bei der Keramik um eine sogenannte Dentalkeramik handelt, wobei vorzugsweise die Dentalkeramik Zirkonoxid (ZrO₂), Yttriumoxid (Y₂O₃), Hafniumoxid (HfO₂) und/oder Aluminiumoxid (Al₂O₃) und ggf. weitere Oxide umfasst.

11. Granulat aus Keramik, Glaskeramik oder Glas, vorzugsweise Keramik, das nach dem Verfahren nach einem der vorhergehenden Ansprüche erhältlich ist und eine Restfeuchte zwischen 5 und weniger als 20 %, insbesondere zwischen 5 und 15 %, aufweist.

12. Formkörper, insbesondere dentaler Formkörper, hergestellt aus dem Granulat nach Anspruch 11.

13. Formkörper nach Anspruch 12, **dadurch gekennzeichnet, dass** es sich um einen ungesinterten oder nicht endgesinterten Rohling für die Herstellung von Zahnersatz, insbesondere von vollkeramischem Zahnersatz, handelt.

## Claims

1. Method of doping or of colouring ceramic, glass ceramic or glass, in particular in the production of shaped bodies which consist at least partly of these materials, wherein the ceramic, the glass ceramic or the glass is provided as granular material and this granular material is sprayed with a solution which comprises metal ions and/or metal complexes and wherein pores and/or capillaries are present in the granular material and the solution penetrates into these pores and/or capillaries.

2. Method according to claim 1, **characterized in that** the granular material treated with the solution is dried, preferably at a temperature < 100°C.

3. Method according to any one of the previous claims, **characterized in that** the granular material is moved, in particular circulated or stirred, during the spraying and/or drying.

4. Method according to any one of the previous claims, **characterized in that** the solution comprises metal ions or metal complexes of the rare earth elements, in particular of the lanthanoids, or the subgroup elements of the periodic table of the elements.

5. Method according to claim 4, **characterized in that** the solution comprises metal ions or metal complexes of at least one of the elements iron, chromium, copper, yttrium, praseodymium, cobalt, nickel, manganese, erbium or cerium.

6. Method according to any one of the previous claims, **characterized in that** the solution comprises at least one polar solvent, wherein the polar solvent is preferably water or at least one aliphatic alcohol.

7. Method according to any one of the previous claims, **characterized in that** the metal ions or metal complexes are comprised in the solution in a concentration of between 0.01 wt.-% and 70 wt.-%, in particular between 0.1 wt.-% and 50 wt.-%.

8. Method according to any one of the previous claims, **characterized in that** the granular material is further processed to form a shaped body, preferably by means of at least one pressing step, in particular dry pressing step, after the spraying, and optionally after the drying.

9. Method according to any one of the previous claims, **characterized in that** the material is a ceramic, preferably a ceramic based on zirconium oxide (ZrO₂) or aluminium oxide (Al₂O₃).

10. Method according to claim 9, **characterized in that** the ceramic is a so-called dental ceramic, wherein the dental ceramic preferably comprises zirconium oxide (ZrO₂), yttrium oxide (Y₂O₃), hafnium oxide (HfO₂) and/or aluminium oxide (Al₂O₃) and optionally further oxides.

11. Granular material of ceramic, glass ceramic or glass, preferably ceramic, obtainable by the method according to any one of the previous claims and having a residual moisture of between 5 and less than 20%, in particular between 5 and 15%.

12. Shaped body, in particular dental shaped body, produced from the granular material according to claim 11.

13. Shaped body according to claim 12, **characterized in that** it is an unsintered or not finally-sintered blank for producing dental prostheses, in particular all-ceramic dental prostheses.

## Revendications

1. Procédé de dopage ou de coloration de céramique, de vitrocéramique ou de verre, notamment lors de la fabrication de corps moulés qui se composent au moins en partie de ces matériaux, sachant que la céramique, la vitrocéramique ou le verre sont préparés sous forme de granulés et que ces granulés sont pulvérisés avec une solution contenant des ions métalliques et/ou des complexes métalliques, et que des pores et/ou des capillaires sont présents dans les granulés, et la solution pénétrant dans ces pores et/ou capillaires.

2. Procédé selon la revendication 1, **caractérisé en ce que** les granulés traités avec la solution sont séchés, de préférence à une température < 100 °C.

3. Procédé selon l'une des revendications précédentes, **caractérisé en ce que** lors de la pulvérisation et/ou du séchage, les granulés sont agités, notamment circulés ou remués.

4. Procédé selon l'une des revendications précédentes, **caractérisé en ce que** la solution contient des ions métalliques ou des complexes métalliques des éléments de terres rares, en particulier des lanthanides, ou des éléments des sous-groupes du tableau périodique des éléments.

5. Procédé selon la revendication 4, **caractérisé en ce que** la solution contient des ions métalliques ou des complexes métalliques d'au moins un des éléments suivants : fer, chrome, cuivre, yttrium, praséodyme, cobalt, nickel, manganèse, erbium ou cer.

6. Procédé selon l'une des revendications précédentes, **caractérisé en ce que** la solution contient au moins un solvant polaire, lequel solvant polaire est de préférence de l'eau ou au moins un alcool aliphatique.

7. Procédé selon l'une des revendications précédentes, **caractérisé en ce que** les ions métalliques ou les complexes métalliques sont contenus dans la solution avec une concentration comprise entre 0,01 % en poids et 70 % en poids, notamment entre 0,1 % en poids et 50 % en poids.

8. Procédé selon l'une des revendications précédentes, **caractérisé en ce qu'**après la pulvérisation et le cas échéant après le séchage, les granulés font l'objet d'un traitement ultérieur pour obtenir un corps moulé, de préférence à l'aide d'au moins une étape de compression, notamment une étape de compression à sec.

9. Procédé selon l'une des revendications précédentes, **caractérisé en ce que** le matériau est une céramique, de préférence une céramique à base d'oxyde de zirconium (ZrO₂) ou d'oxyde d'aluminium (Al₂O₃).

10. Procédé selon la revendication 9, **caractérisé en ce que** la céramique est une céramique dite dentaire, laquelle céramique dentaire comprend de préférence de l'oxyde de zirconium (ZrO₂), de l'oxyde d'yttrium (Y₂O₃), de l'oxyde d'hafnium (HfO₂) et/ou de l'oxyde d'aluminium (Al₂O₃) et éventuellement d'autres oxydes.

11. Granulés de céramique, de vitrocéramique ou de verre, de préférence de céramique, qui peuvent être obtenus avec le procédé selon l'une des revendications précédentes et présentent une humidité résiduelle comprise entre 5 et moins de 20 %, notamment entre 5 et 15 %.

12. Corps moulé, en particulier corps moulé dentaire, réalisé à partir des granulés selon la revendication 11.

13. Corps moulé selon la revendication 12, **caractérisé en ce qu'**il s'agit d'une ébauche non frittée ou n'ayant pas subi le frittage final, destinée à la réalisation de prothèses dentaires, en particulier de prothèses dentaires entièrement en céramique.
